(19) Europäisches Patentamt
European Patent Office
Office européen des brevets

(11) EP 1 600 438 A1

(12) EUROPEAN PATENT APPLICATION
published in accordance with Art. 158(3) EPC

(43) Date of publication:
30.11.2005 Bulletin 2005/48

(21) Application number: 04714501.6

(22) Date of filing: 25.02.2004

(51) Int Cl.⁷: **C07C 319/14**, C07C 51/43, C07C 57/58, C07C 319/28, C07C 323/56 // (C07B53/00, 55:00), C07M7:00

(86) International application number:
PCT/JP2004/002231

(87) International publication number:
WO 2004/076404 (10.09.2004 Gazette 2004/37)

(84) Designated Contracting States:
AT BE BG CH CY CZ DE DK EE ES FI FR GB GR HU IE IT LI LU MC NL PT RO SE SI SK TR
Designated Extension States:
AL LT LV MK

(30) Priority: 28.02.2003 JP 2003054265

(71) Applicant: KANEKA CORPORATION
Osaka-shi, Osaka 530-8288 (JP)

(72) Inventors:
- YAMAMOTO, Shogo
Kobe-shi, Hyogo 655-0872 (JP)
- TAKEDA, Toshihiro
6760075 (JP)
- FUSE, Yoshihide
Kobe-shi, Hyogo 651-2243 (JP)
- UEDA, Yasuyoshi
6711227 (JP)

(74) Representative: Vossius & Partner
Siebertstrasse 4
81675 München (DE)

# (54) PROCESSES FOR THE PRODUCTION OF OPTICALLY ACTIVE COMPOUNDS HAVING SUBSTITUENTS AT THE 2-POSITION

(57) The present invention provides a process for producing an optically active compound having a thio group at the 2-position important for manufacturing medicines. An optically active compound having a hydroxyl group at the 2-position is chlorinated with inversion of the configuration at the 2-position, and the resultant optically active compound having a chlorine atom at the 2-position is reacted with a metal thiolate to introduce a thio group with inversion of the configuration at the 2-position. This process is capable of minimizing racemization and producing an optically active compound having a thio group at the 2-position at low cost in high yield. When the optically active compound having a chlorine atom at the 2-position is reacted with the metal thiolate in coexistence with water in the reaction system, the optically active compound having a thio group at the 2-position with higher optical purity can be produced in higher yield. An optically active carboxylic acid having a thio group at the 2-position is crystallized in the presence of an aliphatic hydrocarbon solvent and/or a sulfur-containing solvent to effectively remove coexistent impurities such as an optical isomer and the like, thereby producing crystals of an optically active carboxylic acid having a thio group at the 2-position with higher purity.



Printed by Jouve, 75001 PARIS (FR)

## Description

Technical Field

The present invention relates to a process for producing optically active compounds having substituents at the 2-positions, the compounds being important as intermediates for producing drugs and the like.

Background Art

Among optically active compounds having substituents at the 2-positions, optically active carboxylic acids having thio groups at the 2-positions, particularly (S)-2-dodecylthiophenylacetic acid, are useful compounds as intermediates for producing drugs, particularly preventives for atherosclerosis (ACAT inhibitors) or hypercholesterolemia (Internal Publication No. 97/19918 pamphlet).

Conventional processes used for producing optically active carboxylic acids having thio groups at the 2-positions include the following:

(i) A chemical optical resolution process using a racemic 2-alkylthiocaboxylic acid (Journal of Organic Chemistry (1967), 32(8), p. 2496-2501).
(ii) A process of alkylthiolating an optically active bromocarboxylic acid prepared by bromination reaction of an optically active amino acid with retention of the configuration (International Publication No. 92/13843 pamphlet).

However, process (i) has a low efficiency of optical resolution and is not necessarily preferable as an industrial production process.

On the other hand, in process (ii), racemization may actively occur in a series of steps starting from bromination reaction to thio group introduction reaction depending on the type of the amino acid used. Therefore, process (ii) is not a general-purpose process for obtaining a high-optical-purity optically active carboxylic acid having a thio group at the 2-position.

In consideration of the above-mentioned problems, an object of the present invention is to provide a high-yield, low-cost, industrially preferable process for producing an optically active compound having a substituent at the 2-position, for example, a compound which is easily racemized, such as an optically active carboxylic acid having a thio group at the 2-position, with minimum racemization.

Disclosure of Invention

As a result of intensive research, the inventors of the present invention have found that a high-yield, low-cost process for producing an optically active compound having a thio group at the 2-position with minimum racemization is only a process in which an optically active compound having a hydroxyl group at the 2-positon is chlorinated with inversion of the configuration at the 2-position, and then the resulting optically active compound having a chlorine atom at the 2-position is reacted with a metal thiolate to introduce a thio group with inversion of the configuration at the 2-position.

It has been also found that when the optically active compound having a chlorine atom at the 2-position is reacted with a metal thiolate to introduce a thio group with inversion of the configuration at the 2-position, the coexistence of water in the reaction system can shorten the reaction time and produce an optically active compound having a thio group at the 2-position with higher optical purity and/or in higher yield.

It has been further found that when a metal salt of an optically active carboxylic acid having a thio group at the 2-position is treated in a mixed solvent system containing water and an ester solvent and/or an ether solvent, the optically active carboxylic acid having a thio group at the 2-postiion can be extracted as the metal salt in an organic layer, and consequently coexistent inorganic compounds such as the metal base and the like used in the reaction can be effectively removed to an aqueous layer. In this case, the acid used for neutralizing the metal salt of the optically active carboxylic acid having a thio group at the 2-position in a subsequent step can be decreased. Thereby the amount of waste, such as an inorganic salt and the like, can be decreased significantly.

It has been further found that when the optically active carboxylic acid having a thio group at the 2-positoin is crystallized as a free acid or a salt with a base using an aliphatic hydrocarbon solvent and/or a sulfur-containing solvent, coexistent impurities such as an optical isomer and the like can be effectively removed to obtain crystals of the optically active carboxylic acid with improved optical purity and/or improved chemical purity.

It has been further found that when an optically active 2-chlorocarboxylic acid is crystallized with an aromatic hydrocarbon solvent and/or an ester solvent, the optically active 2-chlorocarboxylic acid can be obtained as crystals not containing coexistent impurities such as an optical isomer and the like.

The present invention has been completed on the basis of the above findings.

The present invention relates to a process for producing an optically active compound having a thio group at the 2-position and represented by formula (4):

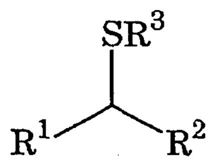

(4)

(wherein $R^1$ represents a substituted or unsubstituted alkyl group having 1 to 6 carbon atoms, a substituted or unsubstituted aryl group having 6 to 14 carbon atoms, or a substituted or unsubstituted aralkyl group having 7 to 15 carbon atoms; $R^2$ represents carboxyl, alkyloxycarbonyl, aralkyloxycarbonyl, halogenated acyl, or nitrile; and $R^3$ represents a substituted or unsubstituted alkyl group having 1 to 15 carbon atoms, a substituted or unsubstituted aryl group having 6 to 14 carbon atoms, or a substituted or unsubstituted aralkyl group having 7 to 15 carbon atoms), the process including chlorinating an optically active compound having a hydroxyl group at the 2-position and represented by formula (1):

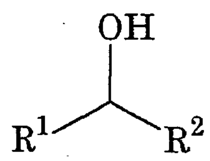

(1)

(wherein $R^1$ and $R^2$ represent the same as the above) with inversion of the configuration at the 2-position to convert the optically active compound (1) to an optically active compound having a chlorine atom at the 2-position and represented by formula (2):

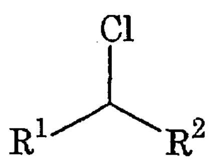

(2)

(wherein $R^1$ and $R^2$ represent the same as the above), and then reacting the optically active compound (2) having a chlorine atom at the 2-position with a metal thiolate represented by formula (3):

$$MSR^3 \qquad (3)$$

(wherein $R^3$ represents the same as the above, and M represents an alkali metal or an alkaline earth metal) to introduce a thio group with inversion of the configuration at the 2-position.

The present invention also relates to a process for producing an optically active compound having.a thio group at the 2-position and represented by formula (4)

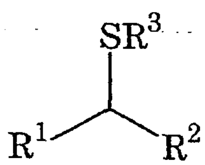

(4)

(wherein $R^1$, $R^2$, and $R^3$ represent the same as the above), the process including reacting an optically active compound (2) having a chlorine atom at the 2-position and represented by formula (2):

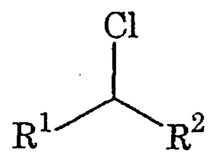

(2)

(wherein $R^1$ and $R^2$ represent the same as the above) with a metal thiolate represented by formula (3):

$$MSR^3 \quad (3)$$

(wherein $R^3$ and M represent the same as the above) in the presence of water to introduce a thio group with inversion of the configuration at the 2-position.

The process may include treating a metal salt of an optically active carboxylic acid having a thio group at the 2-position and represented by formula (5):

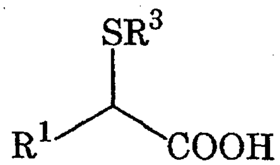

(5)

(wherein $R^1$ and $R^3$ represent the same as the above) in a mixed solvent system containing water and an ester solvent and/or an ether solvent to extract or distribute the metal salt of the optically active carboxylic acid having a thio-group as the 2-postiion in an organic layer.

The present invention further relates to a process for producing (isolating and purifying) an optically active carboxylic acid having a thio group at the 2-position, the process including crystallizing an optically active carboxylic acid having a thio group at the 2-position, containing at least its enantiomer as an impurity, and represented by formula (5):

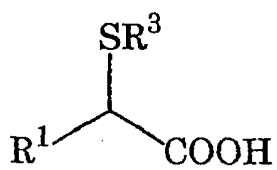

(5)

(wherein $R^1$ and $R^3$ represent the same as the above) with a solvent including an aliphatic hydrocarbon solvent and/or a sulfur-containing solvent.

The present invention further relates to a process for producing (isolating and purifying) an optically active 2-chlorocarboxylic acid represented by formula (6):

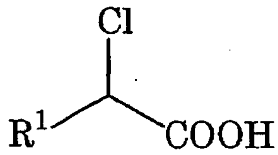

(6)

(wherein $R^1$ represents the same as the above) as crystals with improved optical purity, the process including crystallizing an optically active 2-chlorocarboxylic acid containing at least its optical isomer as an impurity and represented by formula (6) with an aromatic hydrocarbon solvent and/or an ester solvent.

Best Mode for Carrying Out the Invention

The present invention will be described in detail below.

The compounds used in the present invention are represented by formulae (1) to (7), respectively.

In formulae (1), (2), (4), (5), (6), and (7), $R^1$ represents a substituted or unsubstituted alkyl group having 1 to 6 carbon atoms, a substituted or unsubstituted aryl group having 6 to 14 carbon atoms, or a substituted or unsubstituted aralkyl group having 7 to 15 carbon atoms.

Examples of a substituted or unsubstituted alkyl group having 1 to 6 carbon atoms include methyl, ethyl, n-propyl, isopropyl, n-butyl, sec-butyl, tert-butyl, pentyl, and hexyl. An alkyl group having 1 to 4 carbon atoms is preferred.

Examples of a substituted or unsubstituted aryl group having 6 to 14 carbon atoms include phenyl, p-hydroxyphenyl, naphthyl, and biphenyl. An aryl group having 6 to 8 carbon atoms is preferred, and a phenyl group is more preferred.

Examples of a substituted or unsubstituted aralkyl group having 7 to 15 carbon atoms include benzyl, p-chlorobenzyl, p-hydroxybenzyl, p-fluorobenzyl, m,m-difluorobenzyl, and phenylethyl.

An aralkyl group having 7 to 8 carbon atoms is preferred, and a benzyl group is more preferred.

Examples of a substituent of alkyl, aryl, or aralkyl as $R^1$ include halogens such as fluorine, chlorine, bromine, and iodine, nitro, nitroso, cyano, amino, hydroxyamino, alkylamino having 1 to 12 carbon atoms, dialkylamino having 2 to 10 carbon atoms, N-protected amino, azido, trifluoromethyl, carboxyl, formyl, acetyl; benzoyl, hydroxyl, alkyl having 1 to 12 carbon atoms, alkyloxy having 1 to 12 carbon atoms, and alkylthio having 1 to 12 carbon atoms. Among these groups, halogens, nitro, N-protected amino, and alkyloxy having 1 to 12 carbon atoms are preferred. The number of substituents of each group is 0 to 3.

Examples of an alkyl moiety having 1 to 12 carbon atoms in a substituent, such as alkylamino having 1 to 12 carbon atoms, alkyl having 1 to 12 carbon atoms, alkyloxy having 1 to 12 carbon atoms, or alkylthio having 1 to 12 carbon atoms, include methyl, ethyl, propyl, butyl, pentyl, hexyl, heptyl, octyl, nonyl, decyl, undecyl, and dodecyl. In dialkylamino having 2 to 10 carbon atoms, an alkyl group is preferably selected so that the total carbon number of two alkyl groups is 2 to 10.

Examples of a protective group of the N-protected amino group include the protective groups described in Protective Groups in Organic Synthesis, 2nd Ed., written by Teodora W. Green and published by JOHN WILEY & SONS, 1990, pp. 309-384. Specific examples of the protective group include aralkyl protective groups, such as benzyl, phenylethyl, and triphenylmethyl; sulfonyl protective groups, such as methanesulfonyl, trifluoromethanesulfonyl, benzenesulfonyl, p-toluenesulfonyl, o-nitrobenzenesulfonyl, m-nitrobenzenesulfonyl, and p-nitrobenzenesulfonyl; carbamate protective groups, such as methoxycarbonyl, ethoxycarbonyl, tert-butoxycarbonyl, and benzyloxycarbonyl; and acetyl protective groups, such as phthaloyl, acetyl, chloroacetyl, trifluoroacetyl, pivaloyl, and benzoyl.

$R^1$ is preferably a substituted or unsubstituted aryl group having 6 to 14 carbon atoms, more preferably an aryl group having 6 to 8 carbon atoms, and further preferably phenyl.

In formulae (1), (2), and (4), $R^2$ is, for example, carboxyl; alkyloxycarbonyl, such as methyloxycarbonyl, ethyloxycarbonyl, n-propyloxycarbonyl, isopropyloxycarbonyl, or butyloxycarbonyl; aralkyloxycarbonyl, such as benzyloxycarbonyl; halogenated acyl, such as chloroformyl or bromoformyl; or nitrile.

$R^2$ is preferably carboxyl.

In formulae (3), (4), and (5), $R^3$ is a substituted or unsubstituted alkyl group having 1 to 15 carbon atoms, a substituted or unsubstituted aryl group having 6 to 14 carbon atoms, or a substituted or unsubstituted aralkyl group having 7 to 15 carbon atoms.

Examples of a substituted or unsubstituted alkyl group having 1 to 15 carbon atoms as $R^3$ include methyl, ethyl, propyl, butyl, pentyl, hexyl, heptyl, octyl, nonyl, decyl, undecyl, dodecyl, tridecyl, and pentadecyl. $R^3$ is preferably alkyl having 4 to 12 carbon atoms, more preferably alkyl having 6 to 12 carbon atoms, and further preferably dodecyl.

A substituted or unsubstituted aryl group having 6 to 14 carbon atoms as $R^3$ is exemplified by the same groups as described above for a substituted or unsubstituted aryl group having 6 to 14 carbon atoms as $R^1$. $R^3$ is preferably an aryl group having 6 to 8 carbon atoms and more preferably phenyl.

A substituted or unsubstituted aralkyl group having 7 to 15 carbon atoms as $R^3$ is exemplified by the same as described above for a substituted or unsubstituted aralkyl group having 7 to 15 carbon atoms as $R^1$. $R^3$ is preferably an aralkyl group having 7 to 8 carbon atoms and more preferably benzyl.

$R^3$ is preferably a substituted or unsubstituted alkyl group having 1 to 15 carbon atoms, more preferably an alkyl group having 4 to 12 carbon atoms, further preferably an alkyl group having 6 to 12 carbon atoms, and particularly preferably dodecyl.

In formula (3), M is an alkali metal or an alkaline earth metal.

Examples of an alkaline earth metal include lithium, sodium, and potassium.

Examples of an alkaline earth metal include magnesium and calcium.

M is preferably an alkali metal and particularly preferably potassium.

Description will be made of each step of the present invention.

First, the optically active compound (2) having a chlorine atom at the 2-position can be effectively synthesized by, for example, the chlorination process below. For example, the optically active compound (1) having a hydroxyl group at the 2-position can be chlorinated using thionyl chloride with inversion of the configuration at the 2-position.

The solvent used in the reaction is not particularly limited as long as it is an organic solvent. For example, an ether solvent and/or an aromatic hydrocarbon solvent can be used.

Examples of the ether solvent include, but not limited to, tetrahydrofuran, 1,4-dioxane, and methyl tert-butyl ether.

Examples of the aromatic hydrocarbon solvent include, but not limited to, toluene, and xylene.

The reaction in the presence of a basic compound can synthesize the optically active compound (2) having a chlorine atom at the 2-position in higher yield.

Examples of the basic compound include, but not limited to, N,N-dimethylformamide, and tetrabutylammonium fluoride.

The reaction temperature is not particularly limited, but the temperature is usually -20° C to 100° C, preferably -10° C to 50° C, and more preferably 0° C to 30° C. The reaction time is not particularly limited, but the time is usually 1 to 100 hours, preferably 1 to 48 hours, and more preferably 1 to 24 hours.

The optically active compound (2) having a chlorine atom at the 2-position may be either an (R) isomer or an (S) isomer. For example, an optically active compound having a chlorine atom at the 2-position with an (R) configuration can be synthesized from an optically active compound having a hydroxyl group at the 2-position with an (S) configuration. Similarly, an optically active compound having a chlorine atom at the 2-position with an (S) configuration can be synthesized from an optically active compound having a hydroxyl group at the 2-position with an (R) configuration.

The optically active compound (2) having a chlorine atom at the 2-position is not particularly limited, but the compound (2) is preferably, for example, an optically active 2-chlorocarboxylic acid (6) or the like, and particularly an optically active 2-chlorophenylacetic acid or the like. The optically active 2-chlorbcarboxylic acid (6) can be produced by chlorinating an optically active 2-hydroxycarboxylic acid (7) .

In order to isolate the product from the reaction solution of the optically active compound (2) having a chlorine atom at the 2-position, general post-treatment may be performed. For example, after the reaction, water and an extraction solvent are added to the reaction solution to extract the product into an organic layer.

Examples of the extraction solvent include, but not limited to, toluene, ethyl acetate, methyl tert-butyl ether, and methylene chloride. In particular, toluene and ethyl acetate are preferred, and ethyl acetate is particularly preferably used.

When $R^2$ in formula (2) is a carboxyl group, i.e., in the case of the optically active 2-chlorocarboxylic acid represented by formula (6), in order to improve chemical purity and/or decrease coloring, preferably, an extract or a concentrate thereof is treated with a base to convert the optically active 2-chlorocarboxylic acid (6) to a salt with the base, and the resulting salt is transferred to the aqueous layer and then washed with an organic solvent. The salt of the optically active 2-chlorocarboxylic acid (6) transferred to the aqueous layer can be converted to the optically active free 2-chlorocarboxylic acid (6) by salt dissociation (for example, neutralization with an acid having higher acidity than that of the optically active 2-chlorocarboxylic acid (6), such as a mineral acid, e.g., hydrochloric acid or sulfuric acid). The optically active free 2-chlorocarboxylic acid (6) is extracted into an organic solvent, and if required, the solvent is removed to obtain the optically active 2-chlorocarboxylic acid (6) with improved chemical purity and reduced coloring. The thus-obtained optically active 2-chlorocarboxylic acid (6) with higher purity is used for synthesizing an optically active carboxylic acid (5) having a thio group at the 2- position. In this synthesis, impurities which may cause difficulty in crystallization are previously removed, and thus, high-purity crystals of the optically active carboxylic acid (5) having a thio group at the 2-position can be effectively obtained by crystallizing the optically active carboxylic acid (5) having a thio group at the 2-position.

The salt of the optically active 2-chlorocarboxylic acid (6) with the base may be obtained as an aqueous solution or crystals, and a preferred form can be selected according to purposes.

The base used for the neutralization is not particularly limited, but an alkali metal compound or an alkaline earth metal compound can be used. Specific examples of the base include alkali metal hydroxides, such as lithium hydroxide, sodium hydroxide, and potassium hydroxide; alkaline earth metal hydroxides, such as magnesium hydroxide and calcium hydroxide; alkali metalcarbonates,such aslithium carbonate, sodium carbonate, and potassium carbonate; and alkali metal hydrogen carbonates, such sodium hydrogen carbonate and potassium hydrogen carbonate. Among these compounds, alkali metal hydroxides are preferred, and sodium hydroxide and potassium hydroxide are more preferred.

The neutralization with the base is preferably performed in the presence of water under conditions of weak acidic to basic. The lower limit of pH is usually 4, preferably 5, more preferably 6, and further preferably 7. The upper limit of pH is not particularly limited, but it is usually 14 and preferably 13.

The optically active compound (2) having a chlorine atom at the 2-position and/or the optically active 2-chlorocarboxylic acid (6) may be directly used as an extract or a concentrate thereof. However, purification may be further performed by a general method such as crystallization, distillation, or column chromatography to further improve purity according to demand.

The optically active 2-chlorocarboxylic acid (6) is preferably crystallized to effectively improve the purity (particularly optical purity).

The crystallization process for the optically active 2-chlorocarboxylic acid (6) will be described.

The crystallization process is generally carried out in the presence of a solvent.

Examples of the solvent include, without limitation to, hydrocarbon solvents such as aromatic hydrocarbon solvents and aliphatic hydrocarbon solvents; ester solvents; and ether solvents.

Examples of aromatic hydrocarbon solvents include, without limitation to, aromatic hydrocarbons preferably having 6 to 12 carbon atoms, preferably 6 to 10 carbon atoms, and more preferably 6 to 8 carbon atoms, e.g., benzene, toluene, and xylene. In particular, aromatic hydrocarbons having 7 or 8 carbon atoms, e.g., toluene and xylene, are preferred, and toluene is most preferably used.

Examples of aliphatic hydrocarbon solvents include, without limitation to, aliphatic hydrocarbons preferably having 5 to 12 carbon atoms and more preferably 5 to 8 carbon atoms, e.g., pentane, hexane, heptane, and methylcyclohexane. In particular, aliphatic hydrocarbons having 6 or 7 carbon atoms, e.g., hexane, heptane, and methylcyclohexane, are more preferred.

Examples of ester solvents include, without limitation to, esters preferably having 2 to 8 carbon atoms and more preferably 4 to 6 carbon atoms, e.g., ethyl acetate, n-propyl acetate, isopropyl acetate, n-butyl acetate, and tert-butyl acetate. In particular, ethyl acetate is preferred.

Preferred examples of ether solvents include, without limitation to, acyclic ether solvents. Specific examples of the acyclic ether solvents include methyl tert-butyl ether and dibutyl ether. In particular, methyl tert-butyl ether is preferred.

Among these solvents, aromatic hydrocarbon solvents and ester solvents are preferred, and specifically, toluene and ethyl acetate are preferred.

Of course, the crystallization solvents may be used alone or in combination of two or more.

Examples of the crystallizationprocess include general crystallization processes, such as cooling crystallization, concentration crystallization, crystallization using solvent replacement, crystallization by mixing a poor solvent, and salting out. These processes may be performed alone or in appropriate combination.

The crystallization temperature is not particularly limited, but the temperature is usually 60° C or less, preferably 40° C or less, and more preferably 20° C or less. The lower limit is the solidification temperature of the crystallization solution. The crystallization can be generally carried out at -20° C to 40° C and preferably -10° C to 20° C.

The crystallization time is not particularly limited, but the time is usually 1 to 100 hours, preferably 1 to 48 hours, and more preferably 1 to 24 hours.

Although the crystallization is usually carried out under stirring, stirring strength per unit volume is, but not limited to, 0.05 $kW/m^3$ or more, preferably 0.1 $kW/m^3$ more, and more preferably 0.3 $kW/m^3$ or more for example.

After the crystallization is completed, the resultant crystals can be separated by a general solid-liquid separation process, such as centrifugal separation, pressure filtration, or filtration under reduced pressure.

The optical purity of the optically active 2-chlorocarboxylic acid (6) obtained by the crystallization is preferably 97% ee or more, more preferably 98% ee or more, and further preferably 99% ee or more.

Next, description will be made of the process of introducing a thio group in the optically active compound (2) having a chlorine atom at the 2-position using the metal thiolate (3) with inversion of the configuration at the 2-position to produce the optically active compound (4) having a thio group at the 2-postiion.

In the reaction of the compound (2) to produce the compound (4), the expression "inversion of the configuration at the 2-position" means that when the 2-position in formula (2) has an (S) configuration, the 2-position in formula (4)

has an (R) configuration, and when the 2-position in formula (2) has an (R) configuration, the 2-position in formula (4) has an (S) configuration. The 2-position in formula (2) may have either the (S) configuration or the (R) configuration.

In the present invention, the inversion rate of the configuration is preferably 90% or more, more preferably 95% or more, and further preferably 98% or more.

The inversion rate of the configuration is represented by a ratio (percent) of the enantiomer excess (% ee) of the optically active compound having a thio group at the 2-position and represented by formula (4) to the enantiomer excess (% ee) of the optically active compound having a chlorine atom at the 2-position and represented by formula (2), both compounds having opposite configurations.

The metal thiolate (3) is not particularly limited, but a salt prepared from a thiol compound and a base can be used. Preferred examples of the metal thiolate (3) include lithium thiolate, sodium thiolate, potassium thiolate, and cesium thiolate. In particular, potassium thiolate is preferably used.

The metal thiolate (3) may be prepared by reaction of a thiol compound and a base *in situ.*

In this case, the thiol compound is not particularly limited as long as it is represented by $HSR^3$ (wherein $R^3$ represents the same as the above). Examples of the thiol compound include methylthiol, ethylthiol, propylthiol, butylthiol,pentylthiol,hexylthiol,heptylthiol,octylthiol, decylthiol, dodecylthiol, phenylthiol, and benzylthiol.

Examples of the base include, without limitation to, hydroxides, carbonates, and hydrides of the alkali metals or alkaline earth metals described above for M in formula (3).

Specific examples of the base include alkali metal hydroxides, such as lithium hydroxide, sodium hydroxide, and potassium hydroxide; alkaline earth metal hydroxides, such as magnesium hydroxide and calcium hydroxide; alkali metal carbonates, such as lithium carbonate, sodium carbonate, and potassium carbonate; alkaline earth metal carbonates, such as magnesium carbonate and calcium carbonate; and alkali metal hydrides, such as lithium hydride, sodium hydride, and potassium hydride. Among these bases, alkali metal hydroxides are preferred for improving reactivity of thio group introduction reaction, and potassium hydroxide is more preferred.

In the present invention, the amount of the metal thiolate (3) used is not particularly limited, but the amount is 0.8 to 5 times equivalents and preferably 1 to 2 times equivalents relative to the optically active compound (2) having a chlorine atom in the 2-position.

When the metal thiolate (3) is prepared by reaction between the thiol compound and the base in situ, the amount of the thiol compound used is generally 0.7 to 5 times equivalents, preferably 0.9 to 3 times equivalents, and more preferably 1 to 2 times equivalents relative to the optically active compound (2) having a chlorine at the 2-position.

In this case, the amount of the base used is not particularly limited, but the amount is usually 1 to 10 times equivalents, preferably 1 to 5 time equivalent, and more preferably 1 to 3 times equivalents relative to the optically active compound (2) having a chlorine atom at the 2-position.

In the present invention, the reaction for introducing a thio group by reacting the optically active compound (2) having a chlorine atom at the 2-position with the metal thiolate (3) is preferably performed in the presence of water or a polar organic solvent from the viewpoint of suppression of racemization and improvement in yield.

Preferred examples of the polar organic solvent include, but not limited to, ether solvents, ester solvent, ketone solvents, nitrogen-containing solvents, sulfur-containing solvents, and alcohol solvents.

Examples of the ether solvents include diethyl ether, diisopropyl ether, di-n-butyl ether, dimethoxyethane, diethylene glycol dimethyl ether, tetrahydrofuran, 1,4-dioxane, and methyl tert-butyl ether. Among these solvents, tetrahydrofuran is preferred.

Examples of the ester solvents include formates, such as ethyl formate; acetates, such as methyl acetate, ethyl acetate, n-propyl acetate, isopropyl acetate, n-butyl acetate, and tert-butyl acetate; propionates, such as methyl propionate, ethyl propionate, n-propyl propionate, isopropyl propionate, n-butyl propionate, and isobutyl propionate; and ester solvents, such as γ-butyrolactone. Among these solvents, acetates are preferred, and ethyl acetate is more preferred.

Examples of the ketone solvents include acetone, methyl ketone, diethyl ketone, cyclopentanone, cyclohexanone, and dibutyl ketone. Among these solvents, acetone is preferred.

Examples of the nitrogen-containing solvents include N,N-dimethylformamide, acetamide, formamide, acetonitrile, and propionitrile. Among these solvents, N,N-dimethylformamide is preferred.

Examples of the sulfur-containing solvents include thiols, such as hexylthiol and dodecylthiol; sulfides, such as dihexyl sulfide and didodecyl sulfide; disulfides, such as dihexyl disulfide and didodecyl disulfide. Among these solvents, thiols are preferred, and dodecylthiol is more preferred.

Examples of the alcohol solvents include methanol, ethanol, isopropanol, butanol, ethylene glycol, and methoxy alcohol. Among these solvents, methanol, ethanol, and isopropyl alcohol are preferred.

These solvents may be used alone or in combination of two or more.

In particular, when water or a mixed solvent of water and a polar organic solvents is used, the reaction time can be significantly decreased, and the optically active compound (4) having a thio group at the 2-position can be synthesized with higher optical purity and/or in higher yield.

The amount of the water used is not particularly limited, but the lower limit of a volume ratio of water/ (water + polar organic solvent) is preferably 0.01, more preferably 0.02, further preferably 0.03, and particularly preferably 0.05. The upper limit of the ratio is preferably 0. 90, more preferably 0.70, and further preferably 0.50.

The total of the water and the polar organic solvent used is not particularly limited, but in view of economics or the like, the lower limit of the total is 0.05 part by weight, preferably 0.15 part by weight, more preferably 0.5 part by weight, and further preferably 1 part by weight relative to 1 part by weight of the optically active compound (2) having a chlorine atom at the 2-position. The upper limit is, for example, 100 parts by weight, preferably 30 parts by weight, more preferably 10 parts by weight, further preferably 5 parts by weight.

In the above-mentioned reaction, besides water and the polar organic solvent, another organic solvent may be used according to demand.

Examples of the other solvent include, but not limited to, hydrocarbon solvents, such as benzene, toluene, n-hexane, heptane, cyclohexane, and methylcyclohexane; halogenated solvents, such as methylene chloride, chloroform, 1,1,1-trichloroethane, carbon tetrachloride, 1,2-dichloroethane, and monochlorobenzene. These solvents may be used alone or in combination of two or more.

The reaction temperature is not particularly limited, but the reaction can be usually performed at 60° C or less, preferably 40° C or less, and more preferably 30° C or less. The lower limit of the reaction temperature is the solidification temperature of the system, which is usually -20° C, preferably -10° C, and more preferably 0° C.

The reaction time is not particularly limited, but the time is usually 1 to 100 hours and preferably 1 to 48 hours.

The reaction is generally performed under stirring, and the agitation power per unit volume is not particularly limited. However, the agitation power is usually 0.05 $kW/m^3$ or more.

In order to obtain the product from the reaction solution after the completion of the reaction, general post-treatment may be carried out. For example, the product can be extracted with an organic solvent in coexistence with the water used in the reaction or water separately added after the completion of the reaction as occasion demands.

As the organic solvent for extraction, the organic solvent used in the reaction may be partially or entirely used or the organic solvent may be separately added for the extraction.

Preferred examples of the extraction solvent include, but not limited to, hydrocarbon solvents, such as hexane, heptane, and toluene; halogenated solvents, such as methylene chloride; ester solvents, such as ethyl formate, methyl acetate, ethyl acetate, isopropyl acetate, and butyl acetate; and ether solvents, such as diethyl ether, diisopropyl ether, methyl tert-butyl ether, and tetrahydrofuran.

When $R^2$ in the optically active compound (4) having a thio group at the 2-position is a carboxyl group, i.e., in the case of the optically active carboxylic acid (5)- having a thio group at the 2-position, the compound (5) can be transferred in any desired state, for example, a free acid or a metal salt, to an organic layer by extraction. After the completion of the reaction, the base used in the reaction or the inorganic salt and the like by-produced in the reaction can be removed into an aqueous layer by a simple operation of stirring the reaction solution in a mixed solvent of water and an organic solvent. In this case, the metal salt of the optically active carboxylic acid (5) having a thio group at the 2-position can be dissociated with a minimum amount of an acid, thereby causing an advantage in reducing the salt dissociation time, decreasing the waste, improving productivity at the industrial level, or decreasing environmental loads.

Preferred examples of the organic solvent include, but not limited to, ester solvents, such as ethyl formate, methyl acetate, ethyl acetate, isopropyl acetate, and butyl acetate; and ether solvents, such as diethyl ether, diisopropyl ether, methyl tert-butyl ether, and tetrahydrofuran. In particular, ethyl acetate and tetrahydrofuran are more preferred. These solvents may be used alone or as a mixed solvent of two or more.

The acid used in the salt dissociation reaction is not particularly limited, but, for example, a mineral acid such as hydrochloric acid or sulfuric acid is preferably used. The pH in treatment with the acid is, for example, preferably 0 to 6 and more preferably 1 to 4.

The reaction solvent, the extraction solvent, and water can be distilled off from the extract of the optically active carboxylic acid (5) having a thio group at the 2-position obtained as described above by an operation such as heating under reduced pressure or the like, and thereby the optically active carboxylic acid (5) having a thio group at the 2-position can be obtained.

The extract or concentrate of the optically active carboxylic acid (5) having a thio group at the 2-position is preferably washed with water or an aqueous solution such as brine or solution of sodium sulfate in order to remove coexistent impurities.

The optically active carboxylic acid (5) having a thio group at the 2-position obtained as described above may be crystallized to obtain the optically active carboxylic acid (5) having a thio group at the 2-position as free acid crystals.

The crystallization process of the present invention can be used for obtaining the optically active carboxylic acid (5) having a thio group at the 2-position from the reaction solution and for recrystallizing the carboxylic acid (5). For example, the optically active carboxylic acid (5) having a thio group at the 2-position is obtained as crystals of a salt with a base to remove coexistent impurities, and then the resulting salt of the optically active carboxylic acid (5)

having a thio group at the 2-position is dissociated and then crystallized to obtain the optically active carboxylic acid (5) having a thio group at the 2-position with higher purity.

First, description will be made of the process for crystallizing the optically active carboxylic acid (5) having a thio group at the 2-postiion to produce free acid crystals thereof.

The solvent used in the present invention substantially includes an aliphatic hydrocarbon solvent and/or a sulfur-containing solvent from the viewpoint of removal of the coexistent enantiomer and improvement in crystallization yield. Namely, the solvent may be an aliphatic hydrocarbon solvent, a sulfur-containing solvent, or a mixture of an aliphatic hydrocarbon solvent and a sulfur-containing solvent. The term "substantially" means that a solvent other than the aliphatic hydrocarbon solvent and the sulfur-containing solvent may coexist in a range which has no adverse effect.

The aliphatic hydrocarbon solvent is not particularly limited, but, for example, an aliphatic hydrocarbon having 5 to 12 carbon atoms can be used. Specific examples include pentane, hexane, heptane, octane, decane, undecane, dodecane, cyclohexane, methylcyclohexane, and ethylcyclohexane. Among these solvents, hexane and heptane are preferred.

Examples of the sulfur-containing solvent include, but not limited to, thiol solvents, sulfide solvents, disulfide solvents, and polysulfide solvents. Among these solvents, thiol solvents and disulfide solvents are preferred.

The thiol solvents are not particularly limited, but alkylthiols having 6 to 12 carbon atoms, for example, hexylthiol, heptylthiol, octylthiol, nonylthiol, decylthiol, undecylthiol, and dodecylthiol, can be preferably used. In particular, alkylthiols having 8 to 12 carbon atoms are preferred, and alkylthiols having 10 to 12 carbon atoms are more preferred. Specific examples of such preferred solvents include decylthiol, undecylthiol, and dodecylthiol.

The sulfide solvents are not particularly limited, but dialkyl sulfides having 12 to 24 carbon atoms, for example, dihexyl sulfide, diheptyl sulfide, dioctyl sulfide, dinonyl sulfide, didecyl sulfide, diundecyl sulfide, and didodecyl sulfide, can be used. In particular, dialkyl sulfides having 16 to 24 carbon atoms are preferred, and dialkyl sulfides having 20 to 24 carbon atoms are more preferred. Specific examples of such preferred solvents include didecyl sulfide, diundecyl sulfide, and didodecyl sulfide.

The disulfide solvents are not particularly limited, but dialkyl disulfides having 12 to 24 carbon atoms, for example, dihexyl disulfide, diheptyl disulfide, dioctyl disulfide, dinonyl disulfide, didecyl disulfide, diundecyl disulfide, and didodecyl disulfide, can be used.

In particular, dialkyl disulfides having 16 to 24 carbon atoms are preferred, and dialkyl disulfides having 20 to 24 carbon atoms are more preferred. Specific examples of such preferred solvents include didecyl disulfide, diundecyl disulfide, and didodecyl disulfide.

The polysulfide solvents are not particularly limited, but, for example, trihexyltrisulfide, tridodecyltrisulfide, and polydodecyl polysulfide, can be used.

As the sulfur-containing solvent used as the crystallization solvent, the thiol compound used in the reaction of the present invention or its sulfide compound may be also used as the crystallization solvent. For example, when dodecylthiol is used in the reaction, dodecylthiol, didodecyl sulfide, didodecyl disulfide, or tridodecyl trisulfide, or the like can be used as the crystallization solvent.

The crystallization can be carried out by one or combination of two or more of a general crystallization process, for example, a cooling crystallization process, a concentration crystallization process, a crystallization process using solvent displacement, a crystallization process of mixing a poor solvent, and a salting-out process.

The crystallization temperature is not particularly limited, but the temperature is usually 60° C or less, preferably 40° C or less, and more preferably 20° C or less. The lower limit is the solidification temperature of the crystallization solution, and the crystallization can be preferably carried out at -20 to 40° C and more preferably -10 to 20° C.

The crystallization time is not particularly limited, but the time is usually 1 to 100 hours, preferably 1 to 48 hours, and more preferably 1 to 24 hours.

In the crystallization, a seed crystal can be added to accelerate nucleation according to demand.

The crystallization is preferably performed under stirring, and the agitation power per unit volume is not particularly limited. However, for example, the crystallization is performed under stirring with agitation power 0.05 $kW/m^3$ or more, preferably 0.1 $kW/m^3$ or more, and more preferably 0.3 $kW/m^3$ or more.

After the completion of the crystallization, the resultant crystals can be separated by general solid-liquid separation, such as centrifugal separation, pressure filtration, or filtration under reduced pressure, and, if required, washed with, for example, an aliphatic hydrocarbon solvent (e.g., hexane, heptane, or the like). In washing, the washing solvent may be cooled to a low temperature of 0° C or less in order to decrease a dissolution loss of the precipitated crystals into the washing liquid. The resulting wet crystals of the optically active carboxylic acid (5) having a thio group at the 2-position are dried at, for example, 50° C or less, to obtain dry crystals. Of course, the drying may be performed, for example, under reduced pressure.

The optically active carboxylic acid (5) having a thio group at the 2-position obtained as descried above has an optical purity of 90% ee or more, preferably 95% ee or more, and more preferably 98% ee or more. The optically active carboxylic acid (5) having a thio group at the 2-position may be either an (R) isomer or an (S) isomer.

As descried above, the optically active carboxylic acid (5) having a thio group at the 2-position with high purity can be obtained as free acid crystals. However, if required, the optically active carboxylic acid (5) having a thio group at the 2-position may be obtained as a metal salt or a salt with a base to increase purity before the crystallization of the optically active carboxylic acid (5) having a thio group at the 2-position.

Next, description will be made of the process of crystallizing the optically active carboxylic acid (5) having a thio group at the 2-position as a salt with a metal or a base.

The salt of the optically active carboxylic acid (5) having a thio group at the 2-position with a metal or a base is not particularly limited. Examples of such a salt include metal salts, such as alkali metal salts and alkaline earth metal salts; amine salts, such as alkylamine salts, alkylenediamine salts, aralkylamine salts, arylamine salts, salts with amino-acid esters, and salts with amino-acid amides ; and ammonium salts.

Specific examples of the salt of the optically active carboxylic acid (5) having a thio group at the 2-position include lithium salt, sodium salt, potassium salt, cesium salt, magnesium salt, calcium salt, cyclohexylamine salt, dicyclohexylamine salt, ethylenediamine salt, 1-phenylethylamine salt, 1-(1-naphthyl)ethylamine salt, benzylamine salt, aniline salt, phenylalanine methyl ester salt, phenylalanine ethyl ester salt, phenylglycine methyl ester salt, phenylalanine amide salt, phenylglycine amide salt, and ammonium salt.

Among these salts, alkali metal salts such as potassium salt, alkylamine salts such as cyclohexylamine and dicyclohexylamine salt, and aralkylamine salts such as 1-phenylethylamine salt are preferred.

The base used for converting the optically active carboxylic acid (5) having a thio group at the 2-position to the metal salt or the salt with the base is not particularly limited. Preferred examples of the base include alkali metal hydroxides, alkali metal carbonates, alkali metal hydrogen carbonates, alkaline earth metal hydroxides, alkaline earth metal carbonates, alkylamines, alkylenediamines, aralkylamines, arylamines, amino-acid esters, amino-acid amides, and ammonia.

Specific examples of the base include lithium hydroxide, sodium hydroxide, potassium hydroxide, lithium carbonate, sodium carbonate, potassium carbonate, cesium carbonate, sodium hydrogen carbonate, potassium hydrogen carbonate, magnesium hydroxide, calcium hydroxide, magnesium carbonate, calcium carbonate, cyclohexylamine, dicyclohexylamine, ethylenediamine, 1-phenylethylamine, 1-(1-naphthyl)ethylamine, benzylamine, aniline, phenylalanine methyl ester, phenylalanine ethyl ester, phenylglycine methyl ester, phenylglycine ethyl ester, phenylalanine amide, phenylglycine amide, and ammonia.

Among these bases, alkyl metal hydroxides such as potassium hydroxide, alkylamines such as cyclohexylamine and dicyclohexylamine, and aralkylamines such as 1-phenylethylamine are preferred.

The amount of the base used is 1 to 2 equivalents, preferably 0.8 to 1.4 equivalents, and more preferably 0.8 to 1.2 equivalents relative to the optically active carboxylic acid (5) having a thio group at the 2-position. The base may be added directly or as a solution in another solvent.

The salt of the optically active carboxylic acid (5) having a thio group at the 2-position with the metal of the base can be crystallized by mixing the optically active carboxylic acid (5) having a thio group at the 2-position with the base. More specifically, the crystallization can be carried out by one or combination of two or more of a conventional crystallization process, for example, a cooling crystallization process, a concentration crystallization process, a crystallization process using solvent displacement, a crystallization process of mixing a poor solvent, or a salting-out process. If required, a seed crystal can be added in the crystallization process.

The crystallization process is usually carried out in the presence of a solvent. Examples of the solvent include, but not limited to, hydrocarbon solvents, such as aliphatic hydrocarbon solvents and aromatic hydrocarbon solvents; ester solvents; alcohol solvents; ether solvents; and water.

Examples of aliphatic hydrocarbon solvents include, without limitation to, aliphatic hydrocarbon solvents preferably having 5 to 12 carbon atoms and more preferably 5 to 8 carbon atoms. Specific examples of the aliphatic hydrocarbon solvents include pentane, hexane, heptane, and methylcyclohexane. In particular, aliphatic hydrocarbon solvents having 6 or 7 carbon atoms, e.g., hexane and heptane, are more preferred.

Examples of aromatic hydrocarbon solvents include, without limitation to, aromatic hydrocarbon solvents preferably having 6 to 12 carbon atoms, more preferably 6 to 10 carbon atoms, and further preferably 6 to 8 carbon atoms, e.g., benzene, toluene, and xylene. In particular, aromatic hydrocarbon solvents having 7 or 8 carbon atoms, e.g., toluene and xylene, are preferred, and toluene is most preferably used.

Examples of ester solvents include, without limitation to, esters preferably having 2 to 8 carbon atoms and more preferably 4 to 6 carbon atoms, e.g., ethyl acetate, n-propyl acetate, isopropyl acetate, n-butyl acetate, and tert-butyl acetate. In particular, ethyl acetate is preferred.

Preferred examples of alcohol solvents include, without limitation to, alcohols preferably having 1 to 6 carbon atoms and more preferably 1 to 4 carbon atoms. Specific examples include methanol, ethanol, n-propanol, isopropanol, and butanol. In particular, methanol is preferred.

Preferred examples of ether solvents include, without limitation to, acyclic ether solvents. Specific examples of the acyclic ether solvents include methyl tert-butyl ether and dibutyl ether.

These solvents may be used alone or as a mixed solvent of two or more.

The crystallization temperature is not particularly limited, but the temperature is usually 60° C or less, preferably 40° C or less, and more preferably 20° C or less. The lower limit is the solidification temperature of the crystallization solution. The crystallization can be suitably carried out at -20° C to 40° C and preferably -10° C to 20° C.

The crystallization time is not particularly limited, but the time is preferably 1 to 100 hours, more preferably 1 to 48 hours, and most preferably 1 to 24 hours.

In the crystallization, a seed crystal can be added to accelerate nucleation according to demand.

The crystallization is preferably performed under stirring, and the agitation power per unit volume is not particularly limited. However, for example, the crystallization is performed under stirring with agitation power of 0.05 kW/$m^3$ or more, preferably 0.1 kW/$m^3$ or more, and more preferably 0.3 kW/$m^3$ or more.

The metal salt of the optically active carboxylic acid (5) having a thio group at the 2-position or the salt thereof with the base, which is crystallized by the above crystallization process, can be separated as crystals by a separation operation such as pressure filtration or centrifugal separation. The metal salt of the optically active carboxylic acid (5) having a thio group at the 2-position or the salt thereof with the base may be washed with the organic solvent or water for increasing purity.

The salt of the optically active carboxylic acid (5) having a thio group at the 2-position with the metal or the base, which is produced as described above, is dissociated (for example, neutralized with an acid having higher acidity than that of the optically active carboxylic acid (5) having a thio group at the 2-position, such as a mineral acid, e.g., hydrochloric acid or sulfuric acid) to convert the salt to the optically active free carboxylic acid (5) having a thio group at the 2-position. The optically active free carboxylic acid (5) having a thio group at the 2-position is extracted with an organic solvent and, if required, the solvent is removed to obtain a concentrate or a solution of the optically active carboxylic acid (5) having a thio group at the 2-position.

Preferred examples of the solvent used in the extraction include, but not limited to, hydrocarbon solvents, such as aliphatic hydrocarbon solvents, aromatic hydrocarbon solvents, halogenated hydrocarbon solvents; ester solvents; alcohol solvents; and ether solvents.

Examples of aliphatic hydrocarbon solvents include, without limitation to, aliphatic hydrocarbon solvents preferably having 5 to 12 carbon atoms and more preferably 5 to 8 carbon atoms, e.g., pentane, hexane, heptane, and methylcyclohexane. In particular, aliphatic hydrocarbon solvents having 6 or 7 carbon atoms, e.g., hexane, heptane, and methylcyclohexane, are preferably used.

Examples of aromatic hydrocarbon solvents include, without limitation to, aromatic hydrocarbon solvents preferably having 6 to 12 carbon atoms, more preferably 6 to 10 carbon atoms, and further preferably 6 to 8 carbon atoms, e.g., benzene, toluene, and xylene. In particular, toluene is most preferably used.

Examples of halogenated hydrocarbon solvents include, without limitation to, halogenated hydrocarbon solvents preferably having 1 to 6 carbon atoms and more preferably 1 to 4 carbon atoms. Specifically, chlorinated hydrocarbon solvents having 1 to 4 carbon atoms are further preferred. In particular, methylene chloride is most preferably used.

Examples of ester solvents include, without limitation to, esters preferably having 2 to 8 carbon atoms and more preferably 4 to 6 carbon atoms, e.g., ethyl acetate, n-propyl acetate, isopropyl acetate, n-butyl acetate, and tert-butyl acetate. In particular, ethyl acetate is preferred.

Preferred examples of alcohol solvents include, without limitation to, alcohols preferably having 1 to 6 carbon atoms and more preferably 1 to 4 carbon atoms , e.g., methanol, ethanol, n-propanol, isopropanol, and butanol. In particular, methanol is preferred.

Preferred examples of ether solvents include, without limitation to, acyclic ether solvents. Specific examples of the acyclic ether solvents include methyl tert-butyl ether and dibutyl ether.

Of course, the acid neutralization (salt dissociation) can be carried out in coexistence with the organic solvent.

When the salt of the optically active carboxylic acid (5) having a thio group at the 2-position with the metal or the base is an amine salt, particularly an optically active amine salt with expensive 1-phenylethylamine, 1-(1-naphthyl) ethylamine, amino-acid ester, or amino-acid amide, the amine is preferably recovered as a free amine by a general method and recycled.

Specifically, the amine salt of the optically active carboxylic acid (5) having a thio group at the 2-position is dissociated to produce an amine-acid salt, and this salt is recovered by solid-liquid separation or extraction with water and then neutralized with a base in a mixed solvent of water and an organic solvent or an organic solvent to extract a free amine in the organic layer. If required, the solvent is removed to obtain the free amine or a solution thereof.

The salt of the optically active carboxylic acid (5) having a thio group at the 2-position with the metal or the base, or the free carboxylic acid (5), which is produced as described above, has an optical purity of 95% ee or more, preferably 97% ee or more, and more preferably 99% ee or more.

When the resulting optically active carboxylic acid (5) having a thio group at the 2-position is recrystallized on the basis of the above-mentioned crystallization process, of course, the optically active carboxylic acid (5) having

a thio group at the 2-position can be obtained as a free acid with higher purity. Specifically, the optically active carboxylic acid (5) having a thio group at the 2-position with an optical purity of 99% ee or more, preferably 99.5% ee or more, and more preferably 99.8% ee or more can be obtained.

A representative example of the optically active carboxylic acid (5) having a thio group at the 2-position which can be produced by the present invention is one in which $R^1$ is preferably a substituted or unsubstituted aryl group having 6 to 14 carbon atoms, more preferably a substituted or unsubstituted phenyl group, and further preferably a phenyl group; $R^3$ is a substituted or unsubstituted alkyl group having 1 to 15 carbon atoms, and more preferably a dodecyl group. Specifically, the optically active carboxylic acid (5) is optically active 2-dodecylthiophenylacetic acid.

As seen from the above description and the description of examples below, the process of the present invention is capable of effectively producing the optically active compound (4) having a thio group at the 2-postiion with high optical purity.

Best Mode for Carrying Out the Invention

Although the present invention will be described in detail below with reference to examples, the present invention is not limited to these examples.

(Example 1) <u>(R)-2-Chlorophenylacetic acid</u>

L-(-)-Mandelic acid (5 g, 32.9 mmol) and N,N-dimethylformamide (0.48 g, 6.6 mmol) were added to 50 g of tetrahydrofuran (THF), and the internal temperature was controlled to 20° C. Next, thionyl chloride (11.7 g, 98.3 mmol) was slowly added to the resultant mixture, followed by stirring at 20° C overnight. Then, 20 ml of water and 50 ml of toluene were added to the reaction solution, and the resulting mixture was stirred for a while. After a separation operation, an aqueous layer was discarded, and the residual organic layer was washed twice with 20 ml of water each and then concentrated to obtain a yellow concentrate (5.37 g of (R)-2-chlorophenylaceticacid, yield 95. 8 %) . The evaluation of optical purity by HPLC analysis showed an optical purity of 92.4% ee.

Then, 100 ml of toluene was added to the concentrate, and the mixture was concentrated to about a half under reduced pressure. Then, 50 ml of toluene was again added to the residue, and the resultant mixture was concentrated to about a half under reduced pressure. The residue was stirred under cooling at an internal temperature of 5° C to crystallize of (R)-2-chlorophenylacetic acid. Thus obtained crystals were filtered off with a Kiriyama funnel and then washed twice with 1 ml of toluene each which was cooled to an internal temperature of -15° C. The resulting wet crystals were dried under reduced pressure to obtain 2.27 g of crystals of (R)-2-chlorophenylacetic acid (yield 42.3%). The evaluation of optical purity by HPLC analysis showed an optical purity of 99.5% ee and a chemical purity of 99.1 area %. $^1$H-NMR (400 MHz, $CDCl_3$) $\delta$ (ppm) 5.37 (1H) 7.34-7.53 (5H, m), 10. 68 (1H)

The quantitative measurement and chemical purity measurement of 2-chlorophenylacetic acid and optical purity evaluation of (R)-2-chlorophenylacetic acid were performed using the following analytical method:

Column: manufactured by Daicel Chemical Industries, Ltd. (Chiralpak AD 250x4.6 mm) × 2
Mobile phase : hexane/isopropanol/trifluoroacetic acid = 95/5/0.1
Flow.rate: 0.5 ml/min
Detection: UV 210 nm
Column temperature: 10° C
Retention time: R isomer 44.6 minutes, S isomer 51.8 minutes

The chemical purity was determined by the above analytical method according to the following equation:

Chemical purity = (Area of 2-chlorophenylacetic acid/Total area of all detected compounds) × 100 (area %)

(Example 2) <u>(R)-2-Chlorophenylacetic acid</u>

First, 30 ml of water was added to 109 g of an ethyl acetate solution (colored in light red) of (R)-2-chlorophenylacetic acid (10.9 g, 63.9 mmol, chemical purity 93.8 area %) separately prepared according to the method of Example 1, and the internal temperature was controlled to 20° C. Then, 7.8 g of a 30% aqueous solution of sodiumhydroxide was added to the mixture, followed by stirring for 30 minutes. An aqueous layer was obtained by a separation operation and then washed with 20 ml of ethyl acetate. After the internal temperature was decreased to 5° C, 6.6 g of

35% hydrochloric acid was added to the washings, and the resultant mixture was stirred for 30 minutes. Then, a separation operation was performed to obtain an organic layer. The organic layer was washed with 25 ml of water and then concentrated under reduced pressure to obtain a colorless concentrate containing (R)-2-chlorophenylacetic acid (9.2 g, 53.9 mmol, 92.5% ee) . As a result of evaluation of chemical purity by HPLC analysis, the chemical purity was 95.5 area %.

The resultant concentrate was cooled to an internal temperature of 5° C and allowed to stand at the same temperature to crystallize of (R)-2-chlorophenylaceteic acid. Thus obtained crystals were filtered off with a Kiriyama funnel and then washed twice with 1 ml of ethyl acetate each which was cooled to an internal temperature of -15° C. The resulting wet crystals were dried under reduced pressure to obtain 4.75 g of crystals of (R) -2-chlorophenylacetic acid (yield 51.6%) . The evaluation of optical purity by HPLC analysis showed an optical purity of 98.3% ee and a chemical purity of 99. 5 area %.

(Example 3) <u>(S)-2-Dodecylthiophenylacetic acid</u>

First, 50 ml of THF and 0.48 g (6.6 mmol) of N,N-dimethylformamide were added to L-mandelic acid (5.0 g, 32.9 mmol), and the internal temperature was controlled to 20° C. Next, thionyl chloride (11.7 g, 98.7 mmol) was slowly added to the resultant mixture, followed by stirring at 20° C for 22 hours. Then, 20 ml of water was added to terminate reaction, and 100 ml of toluene was added to the reaction solution. After a separation operation, an aqueous layer was discarded, and the residual organic layer was washed twice with 10 ml of water each and then concentrated to distill off the solvent, thereby obtaining a concentrate of (R)-2-chlorophenylacetic acid (5.05 g, 29.6 mmol, yield 90%, 94% ee). Then, 10.2 g (74.0 mmol) of potassium carbonate and 10.6 ml (44.4 mmol) of 1-dodecylthiol were added to 100 ml of 1,4-dioxane, and then the concentrate of (R)-2-chlorophenylacetic acid was slowly added to the resultant mixture, followed by stirring at 20° C for 40 hours. As a result, (S)-2-dodecylthiophenylacetic acid (7.96 g, 23.7 mmol, yield 80%) with an optical purity 90% ee (configuration inversion rate of 95.77%) and a chemical purity of 93.4 area % was obtained.

The quantitative measurement and chemical purity measurement of 2-dodecylthiophenylacetic acid and the optical purity evaluation of (S)-2-dodecylthiophenylacetic acid were performed using the following analytical method:

Column: manufactured by Daicel Chemical Industries, Ltd. (Chiralpak AD 250×4.6 mm) × 2
Mobile phase:hexane/isopropanol/trifluoroacetic acid = 95/5/0.1
Flow rate: 0.5 ml/min
Detection: UV 210 nm
Column temperature: 10° C
Retention time: S isomer 30.9 minutes, R isomer 27.0 minutes

The chemical purity was determined by the above analytical method according to the following equation:

Chemical purity = (Area of 2-dodecylthiophenylacetic

acid/Total area of all detected compounds) × 100 (area %)

(Comparative Example 1) <u>(S)-2-Dodecylthiophenylacetic acid</u>

D-Phenylglycine (19 g, 0.066 mol) and KBr (31.5 g, 0.264 mol) were dissolved in 132 ml of 2.5 M sulfuric acid (0.33 mol), and the resultant solution was cooled to 0° C. Then, a solution of sodium nitrite (9.2 g, 0.132 mol) in 16.5 ml of water was added to the solution. The reaction solution was stirred at 0° C for 30 minutes and then at room temperature for 2 hours. Then, 50 ml of toluene was added to the reaction solution, followed by a separation operation. The resulting aqueous layer was discarded, and the residual organic layer was washed twice with 30 ml of water each and concentrated under reduced pressure to distill off the solvent, thereby obtaining a concentrate of (R)-2-bromophenylacetic acid (6.4 g, 0.030 mol). The resultant (R)-2-bromophenylacetic acid was dissolved in 120 ml of THF, and the solution was cooled to 0° C. Then, potassium hydroxide (5.9 g, 0.090 mol) was added, and 15 minutes after, dodecylthiol (18.0 g, 0.089 mol) was added. The reaction solution was slowly heated to room temperature and then stirred for 20 hours. As a result, (S)-2-dodecylthiophenylacetic acid having an optical purity of 2% ee was obtained in a yield of 36%.

(Example 4) <u>(S)-2-Dodecylthiophenylacetic acid</u>

First, 2.25 g of ethyl acetate and 0.25 g of water were added to 0.20 g of potassium carbonate, and 0.09 g of

dodecylthiol was added to the resultant mixture at about 20° C, followed by stirring for 1 hour. Then, (R) -2-chlorophenylacetic acid (0.25 g, 0.146 mmol, 92. 0% ee) was added, and the resultant mixture was stirred for 20 hours. As a result, (S)-2-dodecylthiophenylacetic acid with a chemical purity of 89.6 area % and an optical purity of 85.1% ee (configuration inversion rate of 91.8%) was obtained in a yield of 92.7%.

(Example 5) (S)-2-Dodecylthiophenylacetic acid

First, 2.25 g of THF and 0.25 g of water were added to 0.20 g of potassium carbonate, and 0.09 g of dodecylthiol was added to the resultant mixture at about 20° C, followed by stirring for 1 hour. Then, (R)-2-chlorophenylacetic acid (0.25 g, 0.146 mmol, 92.0% ee) was added, and the resultant mixture was stirred for 20 hours. As a result, (S)-2-dodecylthiophenylacetic acid with a chemical purity of 92.2 area % and an optical purity of 90.5 % ee (configuration inversion rate of 97.6%) was obtained in a yield of 90%.

(Example 6) (S)-2-Dodecylthiophenylacetic acid

First, 1.15 g of THF, 1.15 g of ethyl acetate, and 0.25 g of water were added to 0.20 g of potassium carbonate, and 0.09 g of dodecylthiol was added to the resultant mixture at about 20° C, followed by stirring for 1 hour. Then, (R)-2-chlorophenylacetic acid'(0.25 g, 0.146mmol, 92.0% ee) was added, and the resultant mixture was stirred for 20 hours. As a result, (S)-2-dodecylthiophenylacetic acid with a chemical purity of 95. area % and an optical purity of 91.8% ee (configuration inversion rate of 99.0%) was obtained in a yield of 92.7%.

(Example 7) (S)-2-Dodecylthiophenylacetic acid

First, 7.1 g (35.2 mmol) of dodecylthiol was added to 30 g of an ethyl acetate solution of 3.0 g of (R)-2-chlorophenylacetic acid (1.6 mmol, 92.7%ee) separately prepared according to the process of Example 1, followed by stirring. After the internal temperature was controlled to 20° C, a solution of 5.8 g of 85% potassium hydroxide in 4.0 g of water was added to the resultant mixture over 2 hours, and the mixture was stirred for 1 hour. After 6 g of water was added, the mixture was stirred for 30 minutes, and an aqueous layer obtained by a separation operation was discarded. Then, 2.0 g of 35% hydrochloric acid was added to the residual organic layer, and the resulting mixture was stirred for 1 hour. The obtained aqueous layer was discarded, and the residual organic layer was washed with 6 g of water. The organic layer was concentrated under reduced pressure to obtain 10 g of a concentrate containing 5.7g (16.9 mmol, yield 96%) of (S)-2-dodecylthiophenylacetic acid. As a result of evaluation of optical purity by HPLC, the optical purity was 91.8% ee (configuration inversion rate of 99.0%).

(Example 8) (S)-2-Dodecylthiophenylacetic acid

First, 20.0 g of heptane was added to 13.3 g (86.5% ee, chemical purity 67.4 area % (2-chlorophenylacetic acid: 3.3 area %, dodecylthiol: 5.76 area %, didodecyl disulfide: 8.8 area %, and other impurities: 14.7 area %)) of a concentrate (THF solution) containing 10.0 g of (S)-2-dodecylthiophenylacetic acid separately prepared. Then, the resultant mixture was concentrated under reduced pressure until the solvent was not distilled off. Then, 20.0 g of heptane was added to the residue, and the resultant mixture was further concentrated twice under reduced pressure until the solvent was not distilled off to obtain 14.2 g of a concentrate of (S)-2-dodecylthiophenylacetic acid. Then, about 10 mg of a crystal of (S) -2-dodecylthiophenylacetic acid, which was separately prepared, was added as a seed crystal to the concentrate to induce crystallization, followed by stirring at 0° C overnight. The resulting crystals were filtered off with a Kiriyama funnel under reduced pressure, and the internal temperature was decreased to -15° C. The resulting wet crystals were dried under reduced pressure at 40° C to obtain 6.7 g (crystallization yield, 67%) of (S)-2-dodecylthiophenylacetic acid. HPLC evaluation showed an optical purity of 94.5% ee and a chemical purity of 99.3 area % (2-chlorophenylaceticacid: undetected, dodecylthiol: undetected, didodecyl disulfide: undetected, and other impurities: 0.7 area %).

(Example 9) (S)-2-Dodecylthiophenylacetic acid

First, 138 g of heptane was added to 250 g (chemical purity 91.5 area % (didodecyl disulfide: 2.8 area %, and other impurities: 5.7 area %)) of an ethyl acetate solution of 77.1 g (0.229 mol, 92.2%ee) of (S) -2-dodecylthiophenylacetic acid separately prepared according to the process in Example 7. Then, the resultant mixture was concentrated under reduced pressure until the solvent was not distilled off. Then, 138 g of heptane was added to the residue, and the resultant mixture was further concentrated twice under reduced pressure until the solvent was no longer distilled off to obtain 154 g of a concentrate (heptane solution) of (S)-2-dodecylthiophenylacetic acid. The concentrate was

heated to 40° C and then slowly cooled to an internal temperature of 33° C. Then, about 10 mg of a crystal of (S)-2-dodecylthiophenylacetic acid, which was separately prepared, was added as a seed crystal to the concentrate to induce crystallization. The temperature was kept for 30 minutes and then slowly decreased to an internal temperature of 5° C. After further stirring for 1 hour, the resulting crystals were filtered off with a Kiriyama funnel under reduced pressure and washed with 46 ml of heptane cooled to an internal temperature of -15° C and then washed with 23 ml of the cooled heptane. The liquid was sufficiently removed to obtain 64 g of wet crystals. The resulting wet crystals were dried under reduced pressure at 40° C to obtain 63 g (crystallization yield, 81%) of (S)-2-dodecylthiophenylacetic acid crystals. HPLC evaluation showed an optical purity of 98.9% ee and a chemical purity of 99.7 area % (didodecyl disulfide: undetected, and other impurities: 0.3 area %).
$^1$H-NMR (400 MHz, $CDCl_3$) δ (ppm) 0.88 (3H, t, J=6.6Hz), 1.10-1.63 (20H, m), 2.56 (2H, tq, J = 12.0 Hz, 6.8 Hz), 4.56 (1H, s), 7.26-7.48 (5H, m)

(Example 10) (S)-2-Dodecylthiophenylacetic acid

First, 391.1 g of a concentrate (didodecyl disulfide solution) containing 95.6 g (0.284 mol, 92.2% ee, chemical purity 80.7 area % (didodecyl disulfide: 15. 5 area % and other impurities: 3.8 area %)) of (S)-2-dodecylthiophenylacetic acid separately prepared according to the process in Example 7 was controlled to an internal temperature of 30° C and then slowly cooled an internal temperature of 20° C. Then, about 10 mg of a crystal of (S) -2-dodecylthiophenylacetic acid, which was separately prepared, was added as a seed crystal to the concentrate. As a result, precipitation of crystals was confirmed. The temperature was kept for 30 minutes and then slowly decreased to an internal temperature of -5° C, followed by stirring overnight. The resulting crystals were filtered off with a Kiriyama funnel under reduced pressure and washed twice with 23 ml of heptane each which was cooled to an internal temperature of about -15° C. The liquid was sufficiently removed to obtain 73 g of wet crystals. The resulting wet crystals were dried under reduced pressure at 40° C to obtain 72 g (crystallization yield, 75%) of (S)-2-dodecylthiophenylacetic acid crystals. HPLC evaluation showed an optical purity of 98.9% ee and a chemical purity of 99.7 area % (didodecyl disulfide: undetected, and other impurities: 0.3 area %).

(Example 11) (S)-2-Dodecylthiophenylacetic acid dicyclohexylamine salt

First, 0. 490 g (2.7 mmol) of dicyclohexylamine was added to 10 g of a heptane solution containing 1.0g (2.9 mmol, 86. 5%ee, chemical purity 70.0 area % (2-chlorophenylacetic acid: 1.8 area %, dodecylthiol: 6.0 area %, didodecyl disulfide: 9.1 area % and other impurities: 13.1 area %)) of (S)-2-dodecylthiophenylacetic acid separately prepared, followed by stirring. After the internal temperature was controlled to 20° C, the resultant mixture was stirred overnight to crystallize. Thus obtained crystals were filtered off with a Kiriyama funnel under reduced pressure and washed twice with 1 ml of heptane which was cooled to an internal temperature of -15° C to obtain 0.712 g of wet crystals. The resulting wet crystals were dried under reduced pressure at 40° C to obtain 0.700 g (crystallization yield, 70.0%) of wet crystals of (S)-2-dodecylthiophenylacetic acid dicyclohexylamine salt. HPLC evaluation showed an optical purity of 99.9% ee or more and a chemical purity of 98.9 area % (2-chlorophenylacetic acid: undetected, dodecylthiol: undetected, didodecyl disulfide: undetected, and other impurities: 1.1 area %).
$^1$H-NMR (400 MHz, $CDCl_3$ δ (ppm) 0. 88 (3H, t, J= 6.6 Hz), 1.00-1.42, 1.44-1.60, 1.68, 1.87, 2.84 (44H, m), 2.34 (2H, tq, J= 12.3 Hz, 7.3 Hz), 4.46 (1H, s), 7.14-7.54 (5H, m)

The quantitative measurement and chemical purity measurement of 2-dodecylthiophenylacetic acid dicyclohexylamine salt and the optical purity evaluation of (S)-2-dodecylthiophenylacetic acid dicyclohexylamine salt were performed using the following analytical method:

Column: manufactured by Daicel Chemical Industries, Ltd. (Chiralpak AD 250 × 4.6 mm) × 2
Mobilephase : hexane/isopropanol/trifluoroacetic acid = 95/5/0.1
Flow rate: 0.5 ml/min
Detection: UV 210 nm
Column temperature: 10° C
Retention time: S isomer 30.7 minutes

The chemical purity was determined by the above analytical method according to the following equation:

Chemical purity = (Area of 2-dodecylthiophenylacetic acid/Total area of all detected compounds) × 100 (area %)

Industrial Applicability

The process of the present invention is capable of economically and effectively producing an optically active compound having a thio group at the 2-position with high optical purity, which is important for producing medicines and the like.

## Claims

1. A process for producing an optically active compound having a thio group at the 2-position and represented by formula (4):

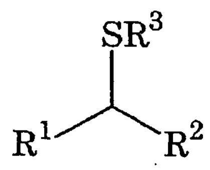

(4)

(wherein $R^1$ represents a substituted or unsubstituted alkyl group having 1 to 6 carbon atoms, a substituted or unsubstituted aryl group having 6 to 14 carbon atoms, or a substituted or unsubstituted aralkyl group having 7 to 15 carbon atoms; $R^2$ represents carboxyl, alkyloxycarbonyl, aralkyloxycarbonyl, halogenated acyl, or nitrile; and $R^3$ represents a substituted or unsubstituted alkyl group having 1 to 15 carbon atoms, a substituted or unsubstituted aryl group having 6 to 14 carbon atoms, or a substituted or unsubstituted aralkyl group having 7 to 15 carbon atoms), the process comprising chlorinating an optically active compound having a hydroxyl group at the 2-position and represented by formula (1):

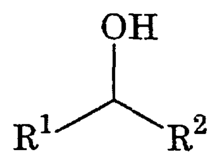

(1)

(wherein $R^1$ and $R^2$ represent the same as the above) with inversion of the configuration at the 2-position to convert the optically active compound (1) to an optically active compound having a chlorine atom at the 2-position and represented by formula (2):

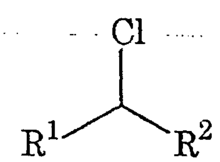

(2)

(wherein $R^1$ and $R^2$ represent the same as the above), and then reacting the optically active compound (2) having a chlorine atom at the 2-position with a metal thiolate represented by formula (3):

$$MSR^3 \qquad (3)$$

(wherein $R^3$ represents the same as the above, and M represents an alkali metal or an alkaline earth metal) to introduce a thio group with inversion of the configuration at the 2-position.

2. The process according to claim 1, wherein $R^1$ is a substituted or unsubstituted phenyl group.

3. The process according to claim 1 or 2, wherein $R^2$ is a carboxyl group.

4. The process according to any one of claims 1 to 3, wherein $R^3$ is a substituted or unsubstituted alkyl group having 1 to 15 carbon atoms.

5. The process according to any one of claims 1 to 4, wherein $R^1$ is a phenyl group, $R^2$ is a carboxyl group, and $R^3$ is a dodecyl group.

6. A process for producing an optically active compound having a thio group at the 2-position and represented by formula (4) :

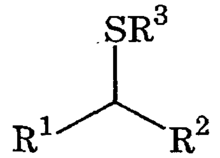

(4)

(wherein $R^1$ represents a substituted or unsubstituted alkyl group having 1 to 6 carbon atoms, a substituted or unsubstituted aryl group having 6 to 14 carbon atoms, or a substituted or unsubstituted aralkyl group having 7 to 15 carbon atoms; $R^2$ represents carboxyl, alkyloxycarbonyl, aralkyloxycarbonyl, halogenated acyl, or nitrile; and $R^3$ represents a substituted or unsubstituted alkyl group having 1 to 15 carbon atoms, a substituted or unsubstituted aryl group having 6 to 14 carbon atoms, or a substituted or unsubstituted aralkyl group having 7 to 15 carbon atoms), the process comprising reacting an optically active compound (2) having a chlorine atom at the 2-position and represented by formula (2):

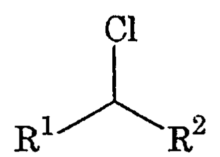

(2)

(wherein $R^1$ and $R^2$ represent the same as the above) with a metal thiolate represented by formula (3):

$$MSR^3 \qquad (3)$$

(wherein $R^3$ the same as the above and M represents an alkali metal or an alkaline earth metal) in the presence of water to introduce a thio group with inversion of the configuration at the 2-position.

7. The process according to claim 6, wherein a polar organic solvent is further used.

8. The process according to claim 7, wherein the polar organic solvent is at least one selected from the group consisting of ester solvents, ether solvents, ketone solvents, nitrogen-containing solvents, sulfur-containing solvents, and alcohol solvents.

9. The process according to claim 7 or 8, wherein the amount of the water used is 0.01 to 0.90 in terms of a volume ratio of water/(water + polar organic solvent).

10. The process according to any one of claims 6 to 9, wherein M of the metal thiolate represented by formula (3) is potassium.

11. The process according to any one of claims 6 to 10, wherein when $R^2$ in formula (4) is a carboxyl group, a metal salt of the produced optically active carboxylic acid having a thio group at the 2-position is treated in a mixed solvent system containing water and an ester solvent and/or water and an ether solvent to extract or distribute the

metal salt of the optically active carboxylic acid having a thio group at the 2-position in an organic layer.

**12.** The process according to claim 11, wherein the ester solvent and/or the ether solvent is ethyl acetate and/or tetrahydrofuran.

**13.** The process according to any one of claims 6 to 12, wherein the introduction of a thio group with inversion of the configuration at the 2-position has a configuration inversion rate of 90% or more.

**14.** The process according to any one of claims 6 to 13, wherein the optically active compound (2) having a chlorine atom at the 2-position and represented by formula (2) is produced by chlorinating an optically active compound having a hydroxyl group at the 2-postiion and represented by formula (1) :

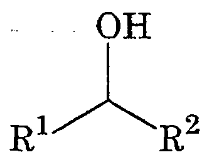

(1)

(wherein $R^1$ and $R^2$ represent the same as the above) with inversion of the configuration at the 2-position.

**15.** The process according to any one of claims 6 to 14, wherein $R^1$ is a substituted or unsubstituted phenyl group.

**16.** The process according to any one of claims 6 to 15, wherein $R^2$ is a carboxyl group.

**17.** The process according to any one of claims 6 to 16, wherein $R^3$ is a substituted or unsubstituted alkyl group having 1 to 15 carbon atoms.

**18.** The process according to any one of claims 6 to 17, wherein $R^1$ is a phenyl group, $R^2$ is a carboxyl group, and $R^3$ is a dodecyl group.

**19.** A process for producing an optically active carboxylic acid having a thio group at the 2-position, the process comprising crystallizing an optically active carboxylic acid having a thio group at the 2-position, containing at least its optical isomer as an impurity, and represented by formula (5):

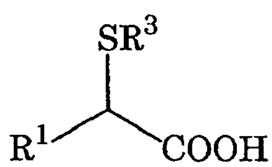

(5)

(wherein $R^1$ represents a substituted or unsubstituted alkyl group having 1 to 6 carbon atoms, a substituted or unsubstituted aryl group having 6 to 14 carbon atoms, or a substituted or unsubstituted aralkyl group having 7 to 15 carbon atoms; and $R^3$ represents a substituted or unsubstituted alkyl group having 1 to 15 carbon atoms, a substituted or unsubstituted aryl group having 6 to 14 carbon atoms, or a substituted or unsubstituted aralkyl group having 7 to 15 carbon atoms) using a solvent including an aliphatic hydrocarbon solvent and/or a sulfur-containing solvent.

**20.** The process according to claim 19, comprising crystallizing the optically active carboxylic acid having a thio group at the 2-position as a free acid.

**21.** The process according to claim 19 or 20, wherein the aliphatic hydrocarbon solvent is at least one selected from the group consisting of hexane, heptane, and methylcyclohexane.

**22.** The process according to any one of claims 19 to 21, wherein the sulfur-containing solvent is at least one selected

from the group consisting of alkylthiols, dialkyl sulfides, dialkyl disulfides, and polyalkyl polysulfides.

**23.** The process according to claim 19, comprising crystallizing the optically active carboxylic acid having a thio group at the 2-position as a salt with a base.

**24.** The process according to claim 23, comprising crystallizing the optically active carboxylic acid having a thio group at the 2-position as an amine salt.

**25.** The process according to claim 24, wherein the amine salt of the optically active carboxylic acid having a thio group at the 2-position is an alkylamine salt of the optically active carboxylic acid having a thio group at the 2-position.

**26.** The process according to claim 19, comprising crystallizing the optically active carboxylic acid having a thio group at the 2-position as a metal salt.

**27.** The process according to claim 26, comprising crystallizing the optically active carboxylic acid having a thio group at the 2-position as an alkali metal salt.

**28.** The process according to any one of claims 19 to 27, wherein the optically active carboxylic acid having a thio group at the 2-position and represented by formula (5) is produced by chlorinating an optically active 2-hydroxycarboxylic acid represented by formula (7):

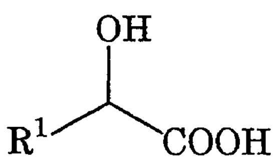

(7)

(wherein $R^1$ represents a substituted or unsubstituted alkyl group having 1 to 6 carbon atoms, a substituted or unsubstituted aryl group having 6 to 14 carbon atoms, or a substituted or unsubstituted aralkyl group having 7 to 15 carbon atoms) with inversion of the configuration at the 2-position, and then reacting the resultant optically active 2-chlorocarboxylic acid represented by formula (6):

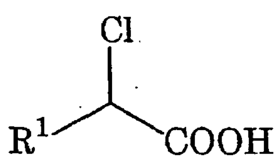

(6)

(wherein $R^1$ represents the same as the above) with a metal thiolate represented by formula (3):

$$MSR^3 \quad (3)$$

(wherein $R^3$ represents the same as the above, and M represents an alkali metal or an alkaline earth metal) to introduce a thio group with inversion of the configuration at the 2-position.

**29.** The process according to claim 28, wherein the optically active 2-chlorocarboxylic acid is improved in chemical purity by a series of operations including transferring the optically active 2-chlorocarboxylic acid as a salt with a base into an aqueous layer, again transferring the optically active 2-chlorocarboxylic acid into an organic solvent layer, and then removing the aqueous layer.

**30.** The process according to any one of claims 19 to 29, wherein $R^1$ is a phenyl group, and $R^3$ is a dodecyl group.

**31.** A process for producing an optically active 2-chlorocarboxylic acid, the process comprising crystallizing an optically active 2-chlorocarboxylic acid containing at least its optical isomer as an impurity and represented by formula (6):

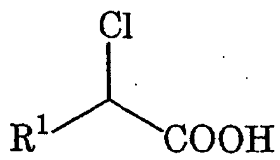

(6)

(wherein $R^1$ represents a substituted or unsubstituted alkyl group having 1 to 6 carbon atoms, a substituted or unsubstituted aryl group having 6 to 14 carbon atoms, or a substituted or unsubstituted aralkyl group having 7 to 15 carbon atoms) using an aromatic hydrocarbon solvent and/or an ester solvent to produce crystals with improved optical purity.

**32.** The process according to claim 31, wherein the aromatic hydrocarbon solvent is toluene.

**33.** The process according to claim 31, wherein the ester solvent is ethyl acetate.

**34.** The process according to any one of claims 31 to 33, wherein the optically active 2-chlorocarboxylic acid to be crystallized is improved in chemical purity by a series of operations including transferring the optically active 2-chlorocarboxylic acid as a salt with a base into an aqueous layer, again transferring the optically active 2-chlorocarboxylic acid into an organic solvent layer, and then removing the aqueous layer.

**35.** The process according to any one of claims 31 to 34, wherein the optically active 2-chlorocarboxylic acid obtained by crystallization has an optical purity of 97% ee or more.

**36.** The process according to any one of claims 31 to 35, wherein the optically active 2-chlorocarboxylic acid represented by formula (6) is produced by chlorinating an optically active 2-hydroxycarboxylic acid represented by formula (7):

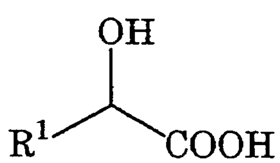

(7)

(wherein $R^1$ represents the same as the above) with inversion of the configuration at the 2-position.

**37.** The process according to any one of claims 31 to 36, wherein $R^1$ is a phenyl group.

## INTERNATIONAL SEARCH REPORT

International application No.
PCT/JP2004/002231

A. CLASSIFICATION OF SUBJECT MATTER
Int.Cl⁷ C07C319/14, 51/43, 57/58, 319/28, 323/56//C07B53/00, 55/00, C07M7:00

According to International Patent Classification (IPC) or to both national classification and IPC

B. FIELDS SEARCHED

Minimum documentation searched (classification system followed by classification symbols)
Int.Cl⁷ C07C319/14, 51/43, 57/58, 319/28, 323/56//C07B53/00, 55/00, C07M7:00

Documentation searched other than minimum documentation to the extent that such documents are included in the fields searched

Electronic data base consulted during the international search (name of data base and, where practicable, search terms used)
CAPLUS(STN), REGISTRY(STN), CASREACT(STN)

C. DOCUMENTS CONSIDERED TO BE RELEVANT

| Category* | Citation of document, with indication, where appropriate, of the relevant passages | Relevant to claim No. |
|---|---|---|
| X | WO 97/19918 A1 (PIERRE FABRE MEDICAMENT), 05 June, 1997 (05.06.97), Full text & US 5990173 A1 | 19-27,30 |
| X | JP 2001-278839 A (Kaneka Corp.), 10 October, 2001 (10.10.01), Full text & EP 1251115 A1 | 31-37 |
| A | WO 92/13843 A1 (PFIZER INC.), 20 May, 1992 (20.05.92), Full text & JP 6-500337 A | 1-18,28,29 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 May, 2004 (25.05.04) | 15 June, 2004 (15.06.04) |
| Name and mailing address of the ISA/ Japanese Patent Office | Authorized officer |
| Facsimile No. | Telephone No. |

Form PCT/ISA/210 (second sheet) (January 2004)

INTERNATIONAL SEARCH REPORT

International application No.
PCT/JP2004/002231

<Concerning the subject of search>

In claims 6-10, 12-15 and 17, compounds wherein $R^2$ is an acyl halide group which is so unstable as to undergoes nucleophilic attack easily are used as ram material or intermediate. However, such compounds having acyl halide groups are not disclosed within the meaning of PCT Article 5 and claim 1 is inadequately supported within the meaning of PCT Article 6.

Therefore, this search has been made about compounds which are disclosed in the description and supported thereby, that is, the compounds wherein $R^2$ is carboxyl, alkyloxycarbonyl, or aralkyloxycarbonyl. Incidentally, complete search has been made on the other claims.

Form PCT/ISA/210 (extra sheet) (January 2004)